# FASCICULE DE BREVET EUROPEEN

(11) **EP 4 259 038 B1**
(45) Date de publication et mention de la délivrance du brevet: **27.11.2024**
(21) Numéro de dépôt: 21851606.0
(22) Date de dépôt: 09.12.2021
(51) Int. Cl.: A61F 2/02, A61F 2/04, A61F 2/48

(54) **DISPOSITIF MEDICAL IMPLANTABLE**
IMPLANTIERBARE MEDIZINISCHE VORRICHTUNG
IMPLANTABLE MEDICAL DEVICE

(30) Priorité: 10.12.2020 FR 2013008
(43) Date de publication de la demande: 18.10.2023
(73) Titulaire: Uromems, 38320 Eybens (FR)
(72) Inventeur: LAMRAOUI, Hamid, 38410 Vaulnaveys le Haut (FR); HO, Thierry, 38120 Saint Egrève (FR)
(74) Mandataire: Regimbeau
(86) Numéro de dépôt international: PCT/FR2021/052248
(87) Numéro de publication internationale: WO 2022/123180

(56) Documents cités:
- WO-A1-2016/083428

## Description

### Domaine technique

L'invention concerne un dispositif médical implantable comprenant un circuit fluidique, destiné à obturer sélectivement un conduit anatomique.

### Etat de la technique

Il existe des dispositifs médicaux implantables dans le corps d'un patient humain ou animal pour pallier un dysfonctionnement d'un organe.

Parmi ces dispositifs médicaux, on peut citer notamment les sphincters urinaires artificiels, qui comprennent une manchette occlusive gonflable adaptée pour être agencée autour d'un conduit naturel tel que l'urètre ou le col vésical, un réservoir de fluide en liaison fluidique avec la manchette, et un actionneur adapté pour transférer du fluide entre la manchette et le réservoir en fonction de la compression à exercer par la manchette sur le conduit naturel.

De tels dispositifs médicaux sont conçus pour fonctionner dans une gamme de pression de fluide donnée, imposée par exemple par une règlementation.

Cependant, il peut exister des situations dans lesquelles la pression évolue hors de cette gamme.

Il est donc souhaitable, même dans ces situations, de prévenir tout dysfonctionnement du patient et d'éviter tout risque pour le patient qui résulterait d'un tel dysfonctionnement.

Le document WO 2016/083428 décrit un dispositif médical implantable selon le préambule de la revendication 1.

### Résumé de l'invention

Un but de l'invention est donc de permettre la prévention d'un dysfonctionnement de l'actionneur pouvant causer un risque pour le patient dans le cas où le patient porteur d'un tel dispositif médical se trouve à une altitude élevée pendant une certaine durée.

Un autre but de l'invention est de permettre la prévention d'un dysfonctionnement de l'actionneur dans le cas où le patient porteur du dispositif médical se trouve sous le niveau de la mer, par exemple lors d'une pratique de la plongée.

A cet effet, l'invention propose un dispositif médical comprenant :
- un boîtier étanche contenant un gaz, adapté pour être implanté dans un corps d'un patient humain ou animal,
- un élément gonflable adapté pour être implanté dans le corps du patient à l'extérieur du boîtier,
- un circuit fluidique comprenant un réservoir de fluide présentant un volume de fluide variable agencé dans le boîtier et une tubulure assurant une liaison fluidique entre ledit réservoir et ledit élément gonflable,
- un actionneur agencé dans le boîtier et couplé mécaniquement au réservoir de fluide pour faire varier sélectivement le volume de fluide dans ledit réservoir,
- une unité de contrôle configurée pour commander l'actionneur de sorte à transférer du fluide entre le réservoir et l'élément gonflable,
- au moins un capteur adapté pour mesurer une pression absolue de fluide dans le circuit fluidique, une force exercée sur le réservoir, une pression de gaz dans le boîtier, une pression atmosphérique ou une valeur relative à une altitude du patient,
ladite unité de contrôle étant en outre configurée pour :
- à partir d'une mesure dudit capteur alors que l'actionneur est inactif, détecter un écart entre une valeur relative à une altitude du patient et une valeur de référence et
- déterminer, en fonction dudit écart, une commande de l'actionneur de sorte à contrôler la pression de fluide dans le circuit fluidique.

Selon des caractéristiques avantageuses mais optionnelles de l'invention, éventuellement combinées :
- l'unité de contrôle est configurée pour, si la valeur relative à l'altitude du patient est supérieure à une première valeur de référence, commander l'actionneur pour réduire le volume de fluide dans l'élément gonflable ;
- l'unité de contrôle est en outre configurée pour maintenir ledit volume réduit de fluide dans l'élément gonflable tant que la valeur relative à l'altitude du patient est supérieure à la première valeur de référence ou que la valeur relative à l'altitude du patient est comprise entre la première valeur de référence et une deuxième valeur de référence inférieure à la première valeur de référence ;
- l'unité de contrôle est configurée pour, si la valeur relative à l'altitude du patient est inférieure à la deuxième valeur de référence, déterminer une commande de l'actionneur pour augmenter le volume de fluide dans l'élément gonflable ;
- l'unité de contrôle est configurée pour, si le patient est immergé et la valeur relative à l'altitude du patient est inférieure à une troisième valeur de référence, commander l'actionneur pour réduire le volume de fluide dans l'élément gonflable ;
- l'unité de contrôle est en outre configurée pour maintenir ledit volume réduit de fluide dans l'élément gonflable tant que le patient est immergé et que la valeur relative à l'altitude du patient est inférieure à la troisième valeur de référence ou que la valeur relative à l'altitude du patient est comprise entre la troisième valeur de référence et une quatrième valeur de référence supérieure à la troisième valeur de référence ;
- l'unité de contrôle est configurée pour, si le patient est immergé et la valeur relative à l'altitude du patient est supérieure à la quatrième valeur de référence, déterminer une commande de l'actionneur pour augmenter le volume de fluide dans l'élément gonflable ;
- l'unité de contrôle est configurée pour déclencher la commande de l'actionneur après l'écoulement d'une durée déterminée pendant laquelle la valeur relative à l'altitude du patient est supérieure à la première valeur de référence ou, si le patient est immergé, la valeur relative à l'altitude du patient est inférieure à une troisième valeur de référence ;
- le réservoir de fluide comprend une partie fixe et une partie mobile et l'actionneur est couplé mécaniquement à ladite partie mobile de sorte à déplacer linéairement ladite partie mobile par rapport à la partie fixe pour faire varier le volume du réservoir ;
- ledit au moins un capteur comprend un capteur agencé dans le boîtier, lié mécaniquement à l'actionneur et/ou à la partie mobile du réservoir, agencé de sorte à mesurer une force de traction et/ou de compression dans la direction de déplacement de la partie mobile du réservoir, ladite force mesurée résultant au moins de :
- la force exercée par la partie mobile du réservoir liée à la pression dans le circuit fluidique, et
- la force exercée sur la partie mobile du réservoir liée à la pression de gaz dans le boîtier,
l'unité de contrôle étant configurée pour mesurer la pression de fluide dans le circuit fluidique à partir de ladite force mesurée ;
- ledit au moins un capteur comprend un capteur de pression de gaz dans le boîtier ;
- ledit au moins un capteur comprend un altimètre agencé dans le boîtier et configuré pour mesurer une valeur relative à l'altitude du patient ;
- le dispositif comprend en outre un dispositif de commande (télécommande) extérieur au corps du patient et adapté pour être porté par le patient, ledit dispositif de commande comprenant un capteur de pression atmosphérique et étant adapté pour communiquer à l'unité de contrôle la pression mesurée par ledit capteur de pression atmosphérique pour déterminer une valeur relative à l'altitude du patient ;
- l'unité de contrôle est configurée pour détecter l'écart d'altitude à partir des données de mesure desdits capteurs de force, de pression ou de l'altimètre ;
- l'unité de contrôle est configurée pour détecter l'écart d'altitude à partir des données de mesure d'un premier capteur parmi lesdits capteurs de force, de pression et l'altimètre et pour confirmer ledit écart d'altitude détecté à partir des données de mesure d'un autre capteur parmi lesdits capteurs de force, de pression et l'altimètre ;
- l'unité de contrôle est configurée pour détecter ledit écart d'altitude de manière discontinue ;
- l'unité de contrôle est configurée pour détecter l'écart d'altitude à des intervalles réguliers compris entre 1 et 300 minutes, de préférence compris entre 5 et 60 minutes, encore de préférence compris entre 5 et 15 minutes ;
- l'élément gonflable est une manchette occlusive adaptée pour être agencée autour d'un conduit anatomique afin d'obturer sélectivement ledit conduit anatomique ;
- le dispositif est configuré pour être implanté dans un corps humain ou animal pour obturer sélectivement au moyen de la manchette, un conduit anatomique choisi parmi au moins : un urètre, un conduit gastrique, un colon et un rectum.

### Brève description des dessins

D'autres caractéristiques et avantages de l'invention ressortiront de la description détaillée qui va suivre, en référence aux dessins annexés, sur lesquels :
- la figure 1 illustre une vue d'ensemble d'un dispositif médical implantable dans le corps d'un patient et d'une télécommande externe au corps du patient.
- la figure 2 est une vue en coupe schématique de l'intérieur du boîtier selon un mode de réalisation.
- la figure 3 est un graphique illustrant de manière schématique des valeurs de référence prises en compte pour le contrôle du dispositif médical lorsque le patient monte en altitude.
- la figure 4 est un graphique illustrant de manière schématique des valeurs de référence prises en compte pour le contrôle du dispositif médical lorsque le patient est immergé.

### Description détaillée de modes de réalisation

Par « patient », on entend dans le présent texte un être humain ou un animal.

La présente invention concerne en particulier les dispositifs médicaux implantables actifs aptes à occlure un conduit naturel, notamment pour lutter contre l'incontinence urinaire au moyen d'un sphincter artificiel capable d'obturer un urètre (chez l'homme) ou le col vésical (chez la femme). Cependant, l'invention concerne de manière plus générale les dispositifs médicaux comprenant un circuit fluidique sensible aux variations de pression notamment générées par des variations d'altitude. Parmi ces autres dispositifs, on peut citer notamment les implants péniens, et les bandes de constriction gastrique.

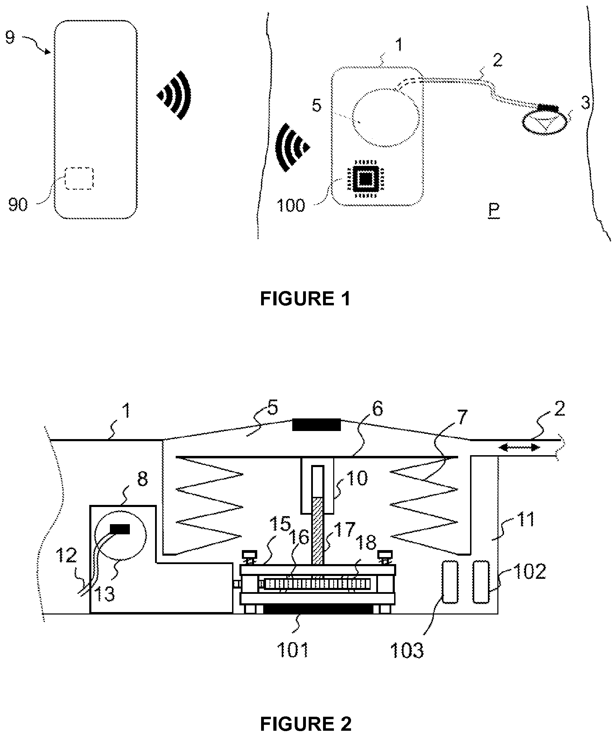

Un dispositif médical implantable dans un corps humain ou animal est illustré à titre d'exemple non limitatif sur les figures 1 et 2.

Le dispositif implantable comprend :
- un boîtier étanche 1 contenant un gaz,
- un élément gonflable 3 adapté pour être implanté dans le corps du patient à l'extérieur du boîtier,
- un circuit fluidique comprenant un réservoir de fluide 5 présentant un volume de fluide variable agencé dans le boîtier et une tubulure 2 assurant une liaison fluidique entre ledit réservoir 5 et ledit élément gonflable 3,
- un actionneur 8 agencé dans le boîtier 1 et couplé mécaniquement à une partie du réservoir de fluide 5 pour faire varier sélectivement le volume de fluide dans ledit réservoir,
- une unité de contrôle 100 configurée pour commander l'actionneur de sorte à transférer du fluide entre le réservoir et l'élément gonflable.

Le circuit fluidique est adapté pour être rempli d'un fluide. Une variation du volume du réservoir 5 entraîne une variation de la pression dans le circuit fluidique. Plus particulièrement, une diminution du volume du réservoir 5 entraîne un transfert de fluide depuis le réservoir 5 vers l'élément gonflable 3, et entraîne une augmentation de la pression dans le circuit fluidique. A l'inverse, une augmentation du volume du réservoir 5 entraîne un transfert de fluide depuis l'élément gonflable 3 vers le réservoir 5, et entraîne une diminution de la pression dans le circuit fluidique.

Le boîtier, la tubulure et la manchette sont adaptés pour être implanté dans le corps d'un patient P, dont les contours sont représentés schématiquement sur la figure 1 de part et d'autre de cet ensemble.

De manière particulièrement avantageuse, une télécommande 9, externe au corps du patient, est utilisable par le patient ou un tiers pour communiquer de manière non filaire avec le dispositif médical.

### Elément gonflable

L'élément gonflable 3 peut être réalisé en matériau biocompatible, tel que du silicone implantable, du polyuréthane implantable, etc. L'élément gonflable 3 peut être réalisé en élastomère biocompatible, par exemple en silicone biocompatible.

L'élément gonflable 3 peut être une manchette occlusive gonflable, en particulier lorsque le système implantable est un sphincter urinaire artificiel. La manchette occlusive gonflable 3 remplie de fluide peut être adaptée pour entourer totalement ou en partie le conduit à occlure.

En variante, l'élément gonflable 3 peut être un implant pénien gonflable, en particulier lorsque le système implantable est une prothèse érectile.

### Liaison fluidique

La liaison fluidique 2 peut consister en une tubulure disposée entre le réservoir 5 et l'élément gonflable 3. Une première extrémité de la tubulure débouche dans le réservoir 5, et une deuxième extrémité de la tubulure débouche dans l'élément gonflable 3.

La liaison fluidique 2 peut être réalisée en matériau biocompatible, tel que du silicone implantable, du polyuréthane implantable, etc. La liaison fluidique 2 peut être réalisée en élastomère biocompatible, par exemple en silicone biocompatible.

### Réservoir à volume variable

Le réservoir 5 comprend une partie fixe par rapport au boîtier et une partie mobile par rapport à la partie fixe, ladite partie mobile étant déplaçable par l'actionneur.

Le réservoir 5 comprend en outre un orifice permettant de transférer le fluide de et à l'extérieur du réservoir 5 vers la manchette 3 via la liaison fluidique 2.

Selon un mode de réalisation avantageux mais non limitatif, la partie fixe du réservoir comprend une partie de la paroi intérieure du boîtier 1, et la partie mobile comprend une paroi mobile 6 de préférence rigide et un soufflet 7 déformable s'étendant entre la paroi mobile 6 et la partie fixe du réservoir.

### Actionneur

L'actionneur 8 peut être adapté pour commander un déplacement linéaire de la paroi mobile 6, le soufflet 7 étant adapté pour s'étendre ou se comprimer en fonction dudit déplacement linéaire de la paroi mobile 6 commandé par l'actionneur 8.

L'actionneur 8 peut être choisi parmi tout système électromécanique permettant de transformer une énergie électrique en un mouvement mécanique avec la puissance requise pour permettre le déplacement à une force et à une vitesse requise de la paroi mobile 6 du réservoir 5 à volume variable. L'actionneur 8 peut notamment être un actionneur piézo-électrique, un actionneur électromagnétique pouvant comprendre un moteur électromagnétique avec ou sans balai couplé ou non à un réducteur, un polymère électro-actif ou un alliage à mémoire de forme.

La paroi mobile 6 peut être déplacée en translation suivant un axe longitudinal par l'action d'une vis d'entraînement 17 solidaire de la paroi mobile 6. La paroi mobile 6 est couplée à la vis d'entraînement 17 par l'intermédiaire d'une noix 10 solidaire de la paroi mobile 6 et présentant un taraudage coopérant avec le filetage de la vis 17. La vis d'entraînement 17 peut s'étendre sensiblement suivant l'axe longitudinal. Une position de la vis d'entraînement 17 peut correspondre sensiblement à un centre de la paroi mobile 6.

L'actionneur 8 est adapté pour entraîner la vis d'entraînement 17 en rotation, par exemple par le biais de la rotation d'un pignon. Une rotation de la vis d'entraînement 17 autour de l'axe longitudinal entraîne un déplacement de la paroi mobile 6 le long de l'axe longitudinal, le soufflet 7 se comprimant ou s'étendant le long de l'axe longitudinal en conséquence.

L'actionneur 8 peut comprendre un moteur 13 couplé à un réducteur. Un connecteur 12 permet d'alimenter le moteur 13 en fonction d'un ordre de fonctionnement du moteur.

Le réducteur est couplé à une roue dentée 18 qui est elle-même couplée à la vis d'entraînement 17, de sorte à transmettre le couple et la rotation de l'axe du moteur 13 à la vis d'entraînement 17. Le système d'entraînement comprend donc une noix couplée à la vis d'entraînement et mobile en rotation sur une butée à billes à double effet autour de l'axe de la vis sous l'effet d'une action d'entraînement par l'actionneur 8, la noix étant couplée à la vis de sorte qu'une rotation de la noix entraîne la vis uniquement en translation dans la direction de déplacement de la partie mobile.

La rotation de la vis 17 entraîne alors la noix en translation, ce qui a pour effet de déplacer la paroi mobile 6 en translation dans une direction parallèle à l'axe de la vis, c'est-à-dire dans la direction de l'axe longitudinal. Le sens de déplacement de la paroi mobile 6 dépend du sens de rotation du moteur 13.

La roue dentée 18 est logée dans un bloc 15 par l'intermédiaire de roulements à billes 16 qui permettent sa rotation dans le bloc 15.

### Unité de contrôle

Le système implantable peut comprendre une unité de contrôle 100 configurée pour recevoir une consigne de pression dans le circuit fluidique, et pour déterminer un volume de réservoir 5 correspondant à la pression de consigne reçue.

L'unité de contrôle peut en outre être adaptée pour commander l'actionneur 8 de sorte à déplacer la paroi mobile 6 du réservoir 5 vers une position correspondant au volume déterminé. Plus particulièrement, dans l'exemple illustré en figure 2, l'unité de contrôle est adaptée pour émettre un ordre de fonctionnement du moteur de l'actionneur 8 électromagnétique dans un sens ou dans l'autre selon qu'une augmentation ou une diminution du volume du réservoir 5 est requise.

### Boîtier

Le réservoir 5 à volume variable et l'actionneur 8 sont intégrés dans un boîtier 1 étanche et biocompatible destiné à être implanté dans le corps du patient. L'unité de contrôle 100 (non illustrée sur la figure 2) peut également être intégrée dans le boîtier 1. Le boîtier renferme également une source d'énergie (non illustrée), par exemple une pile ou une batterie, adaptée pour alimenter électriquement l'unité de contrôle, l'actionneur et les autres composants du dispositif nécessitant une alimentation électrique.

Le boîtier 1, en particulier le volume intérieur 11 du boîtier 1 entourant le réservoir 5, contient un gaz, par exemple un gaz inerte.

Le boîtier 1 peut être réalisé en titane. Le titane étant un matériau étanche et biocompatible, son utilisation permet de protéger les éléments agencés dans le boîtier 1, en particulier les composants électroniques tels que l'unité de contrôle, les éventuels capteurs, etc., de l'environnement extérieur.

Le boîtier 1 est étanche pour éviter tout transfert de fluide ou de gaz de ou vers le milieu intracorporel.

### Ensemble

Un ensemble peut comprendre un dispositif implantable tel que décrit ci-dessus, et une télécommande adaptée pour être utilisée par un individu, par exemple par un individu dans lequel le système est implanté.

Le système implantable et la télécommande comprennent des moyens de communication adaptés pour communiquer entre eux. La télécommande peut être adaptée pour commander un déplacement de la partie mobile du réservoir par l'actionneur 8, en particulier sur la base d'une commande transmise par les moyens de communication de la télécommande aux moyens de communication du dispositif implantable.

Les moyens de communication du dispositif implantable peuvent être intégrés dans le boîtier 1.

### Capteurs

Le boîtier 1 peut renfermer un ou plusieurs capteurs 101, 102 et/ou 103 adaptés pour mesurer une grandeur liée à l'altitude à laquelle se trouve le patient dans lequel le dispositif médical est implanté.

Dans certains modes de réalisation, le boîtier peut comprendre un capteur 101 en liaison mécanique avec l'actionneur 8 et/ou la paroi mobile 6 du réservoir 5 à volume variable. Ledit capteur est adapté pour mesurer une force de compression et/ou de traction dans la direction de déplacement de la partie mobile du réservoir 5, et peut consister en un capteur basé sur une jauge de contrainte, ou un capteur de pression couplé à un mécanisme permettant de mesurer une force. Lorsque l'actionneur est inactif (le volume de fluide étant constant dans le réservoir), une variation de la force mesurée par le capteur traduit une variation de la pression de fluide dans le circuit fluidique.

Dans certains modes de réalisation, un capteur 102 de pression absolue, qui tient compte de la pression de gaz dans le boîtier du dispositif et de la pression atmosphérique, peut être disposé dans le boîtier 1, à l'extérieur du réservoir.

Dans certains modes de réalisation, un capteur de pression atmosphérique 90 peut être agencé dans la télécommande 9 externe au corps du patient.

Dans d'autres modes de réalisation, un altimètre 103 est inclus dans le boîtier 1.

Dans tous les cas, les données de mesure de ces capteurs (capteur de force, de pression absolue, de pression atmosphérique, altimètre) peuvent être reliés à l'altitude du patient. Par exemple, la mesure de la différence de pression entre la pression de gaz dans le boîtier et la pression de fluide dans le réservoir, qui est soumise à la variation de pression atmosphérique, lorsque l'actionneur est inactif, permet de déterminer une valeur d'altitude du patient.

Les données de mesure de chaque capteur sont transmises à l'unité de contrôle qui les traite et les compare à des valeurs de référence pour déterminer s'il est nécessaire ou non de réduire le volume de fluide dans l'élément gonflable. Pour des raisons de lisibilité des figures, les liaisons entre les capteurs et l'unité de contrôle n'ont pas été illustrés. Ces liaisons peuvent être filaires ou non filaires.

Les modes de réalisation décrits ci-dessus peuvent être combinés de sorte à bénéficier des données de mesure de différents capteurs, par exemple pour déterminer de manière plus robuste l'altitude du patient.

Ainsi, par exemple, l'unité de contrôle peut déterminer une valeur d'altitude à partir de données de mesure du capteur de pression 102 et/ou du capteur de force 101 inclus dans le boîtier 1 et ensuite confirmer l'altitude déterminée à partir de données de mesure du capteur de pression atmosphérique 90 de la télécommande 9 disposée hors du corps du patient.

Alternativement, l'unité de contrôle peut déterminer une valeur d'altitude à partir de mesures de la pression atmosphérique par le capteur 90 de la télécommande 9 externe au patient, puis confirmer l'altitude déterminée à partir de données de mesure du capteur de pression 102 et/ou du capteur de force 101 inclus dans le boîtier.

Lorsque l'unité de contrôle détermine un écart entre la valeur relative à l'altitude et une valeur de référence, elle peut activer le dispositif médical afin d'ajuster la pression dans le circuit fluidique. Ainsi, si la valeur relative à l'altitude est telle qu'un risque est encouru pour le fonctionnement du dispositif médical en raison d'une pression élevée dans le circuit fluidique, l'unité de contrôle peut activer l'actionneur pour ouvrir l'élément gonflable, transférant du fluide de l'élément gonflable vers le réservoir, et désactiver l'actionneur tant que la valeur d'altitude n'est pas revenue à une valeur considérée comme acceptable pour le fonctionnement du dispositif.

Pour éviter de désactiver de manière inappropriée le dispositif médical (par exemple si le patient n'est en réalité pas à l'altitude détectée, ou si la durée pendant laquelle il reste à cette altitude est brève), l'unité de contrôle peut être configurée pour confirmer l'altitude déterminée au moyen d'un premier capteur par des données de mesure d'un second capteur, comme expliqué ci-dessus. De manière alternative ou supplémentaire, l'unité de contrôle peut attendre un laps de temps pendant lequel l'altitude déterminée est maintenue, par exemple quelques minutes, avant de commander une réduction du volume de fluide dans l'élément gonflable.

De préférence, les mesures effectuées par les capteurs et les traitements réalisés par l'unité de contrôle ne sont pas réalisés en continu mais à des intervalles réguliers, afin de ne pas consommer trop d'énergie. Ces intervalles peuvent par exemple être compris entre 1 et 300 minutes, de préférence compris entre 5 et 60 minutes, et de manière encore préférée compris entre 5 et 15 minutes.

### Cas d'une montée du patient en altitude

Lorsque le patient monte en altitude, par exemple lors d'un trajet en montagne ou à bord d'un avion, la pression atmosphérique diminue.

Au-delà d'une altitude de l'ordre de 3000 mètres, la pression de fluide dans le circuit fluidique est susceptible de devenir trop élevée pour permettre à l'actionneur de déplacer la partie mobile du réservoir à volume variable et donc d'ouvrir l'élément gonflable.

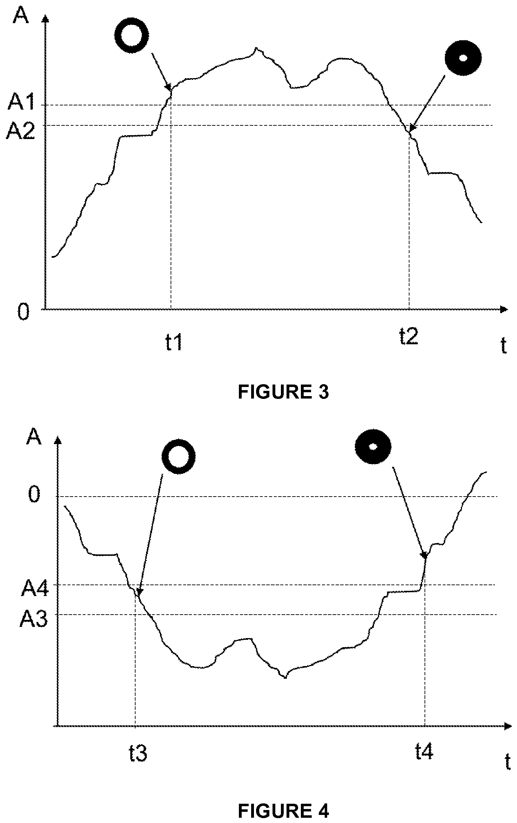

Pour éviter une telle situation, lorsque l'unité de contrôle détecte que le patient se trouve à une altitude supérieure à une première valeur de référence (notée A1 sur le graphique de la figure 3), l'unité de contrôle commande l'actionneur pour ouvrir l'élément gonflable en augmentant le volume du réservoir.

La figure 3 est un graphique illustrant de manière schématique des valeurs de référence prises en compte pour le contrôle du dispositif médical lorsque l'altitude A du patient varie au cours du temps t.

Lorsque le patient monte au-dessus de l'altitude A1, l'unité de contrôle active, à un instant t1 qui est concomitant au passage à l'altitude A1 ou qui le suit de quelques instants (par exemple quelques minutes), l'actionneur pour augmenter le volume du réservoir afin d'ouvrir (c'est-à-dire dégonfler au moins partiellement) l'élément gonflable.

Tant que le patient reste à une altitude supérieure à A1, l'actionneur est désactivé de sorte à conserver l'élément gonflable ouvert (dégonflé).

Une deuxième valeur de référence A2 définit avantageusement une altitude à partir de laquelle l'unité de contrôle peut éventuellement réactiver l'actionneur pour transférer du fluide dans l'élément gonflable.

La valeur A2 est avantageusement choisie inférieure à A1 pour éviter que, si le patient reste pendant une certaine durée à une altitude fluctuant autour de A1, l'unité de contrôle active puis désactive l'actionneur à de multiples reprises pendant une période de temps relativement courte.

Lorsque le patient descend à l'altitude A2, l'unité de contrôle peut activer, à un instant t2 qui est concomitant au passage à l'altitude A2 ou qui le suit de quelques instants (par exemple quelques minutes), l'actionneur pour réduire le volume du réservoir afin de gonfler au moins partiellement l'élément gonflable.

Dans d'autres modes de réalisation, il peut être prévu que l'unité de contrôle désactive l'actionneur jusqu'à ce que le patient se rende chez un praticien qui sera seul habilité à réactiver l'actionneur en vue d'un fonctionnement normal du dispositif médical.

### Cas d'un patient en immersion

Lorsque le patient est immergé sous le niveau de la mer ou d'une autre étendue d'eau, il est soumis à une pression qui croît de l'ordre de 1 bar tous les 10 mètres de profondeur. Pour des raisons de cohérence avec la description qui précède, on considère que la profondeur est une altitude négative en-dessous du niveau de la mer ou de l'étendue d'eau considérée. On se référera donc toujours à une altitude, ladite altitude étant d'autant plus faible que la profondeur d'immersion est grande.

Dans cette situation, le dispositif médical pourrait être endommagé par une surpression dans le circuit fluidique.

La figure 4 est un graphique illustrant de manière schématique des valeurs de référence prises en compte pour le contrôle du dispositif médical lorsque l'altitude A du patient immergé varie au cours du temps t.

Lorsque le patient descend en-dessous de l'altitude A3, l'unité de contrôle active, à un instant t3 qui est concomitant au passage à l'altitude A3 ou qui le suit de quelques instants (par exemple quelques minutes), l'actionneur pour augmenter le volume du réservoir afin d'ouvrir (c'est-à-dire dégonfler au moins partiellement) l'élément gonflable.

Tant que le patient reste à une altitude inférieure à A3, l'actionneur est désactivé de sorte à conserver l'élément gonflable ouvert (dégonflé).

Une deuxième valeur de référence A4 définit avantageusement une altitude à partir de laquelle l'unité de contrôle peut éventuellement réactiver l'actionneur pour transférer du fluide dans l'élément gonflable.

La valeur A4 est avantageusement choisie supérieure à A3 pour éviter que, si le patient reste pendant une certaine durée à une altitude fluctuant autour de A3, l'unité de contrôle active puis désactive l'actionneur à de multiples reprises pendant une période de temps relativement courte.

Lorsque le patient remonte à l'altitude A4, l'unité de contrôle peut activer, à un instant t4 qui est concomitant au passage à l'altitude A4 ou qui le suit de quelques instants (par exemple quelques minutes), l'actionneur pour réduire le volume du réservoir afin de gonfler au moins partiellement l'élément gonflable.

Dans d'autres modes de réalisation, il peut être prévu que l'unité de contrôle désactive l'actionneur jusqu'à ce que le patient se rende chez un praticien qui sera seul habilité à réactiver l'actionneur en vue d'un fonctionnement normal du dispositif médical.

## Revendications

1. Dispositif médical comprenant :
- un boîtier étanche (1) contenant un gaz, adapté pour être implanté dans un corps d'un patient humain ou animal,
- un élément gonflable (3) adapté pour être implanté dans le corps du patient à l'extérieur du boîtier,
- un circuit fluidique comprenant un réservoir de fluide (5) présentant un volume de fluide variable agencé dans le boîtier et une tubulure (2) assurant une liaison fluidique entre ledit réservoir et ledit élément gonflable,
- un actionneur (8) agencé dans le boîtier et couplé mécaniquement au réservoir de fluide pour faire varier sélectivement le volume de fluide dans ledit réservoir,
- une unité de contrôle (100) configurée pour commander l'actionneur de sorte à transférer du fluide entre le réservoir et l'élément gonflable,
- au moins un capteur (101, 102, 103, 90) adapté pour mesurer une pression absolue de fluide dans le circuit fluidique, une force exercée sur le réservoir, une pression de gaz dans le boîtier, une pression atmosphérique ou une valeur relative à une altitude du patient,
ledit dispositif médical étant **caractérisé en ce que** ladite unité de contrôle (100) est en outre configurée pour :
- à partir d'une mesure dudit capteur alors que l'actionneur est inactif, détecter un écart entre une valeur (A) relative à une altitude du patient et une valeur de référence (A1, A2, A3, A4) et
- déterminer, en fonction dudit écart, une commande de l'actionneur (8) de sorte à contrôler la pression de fluide dans le circuit fluidique.

2. Dispositif selon la revendication 1, dans lequel l'unité de contrôle (100) est configurée pour, si la valeur (A) relative à l'altitude du patient est supérieure à une première valeur (A1) de référence, commander l'actionneur pour réduire le volume de fluide dans l'élément gonflable.

3. Dispositif selon la revendication 2, dans lequel l'unité de contrôle (100) est en outre configurée pour maintenir ledit volume réduit de fluide dans l'élément gonflable tant que la valeur (A) relative à l'altitude du patient est supérieure à la première valeur (A1) de référence ou que la valeur (A) relative à l'altitude du patient est comprise entre la première valeur (A1) de référence et une deuxième valeur (A2) de référence inférieure à la première valeur (A1) de référence.

4. Dispositif selon la revendication 3, dans lequel l'unité de contrôle (100) est configurée pour, si la valeur (A) relative à l'altitude du patient est inférieure à la deuxième valeur de référence (A2), déterminer une commande de l'actionneur pour augmenter le volume de fluide dans l'élément gonflable.

5. Dispositif selon l'une des revendications 1 à 4, dans lequel l'unité de contrôle (100) est configurée pour, si le patient est immergé et que la valeur (A) relative à l'altitude du patient est inférieure à une troisième valeur (A3) de référence, commander l'actionneur pour réduire le volume de fluide dans l'élément gonflable.

6. Dispositif selon la revendication 5, dans lequel l'unité de contrôle (100) est en outre configurée pour maintenir ledit volume réduit de fluide dans l'élément gonflable tant que le patient est immergé et que la valeur relative à l'altitude du patient est inférieure à la troisième valeur (A3) de référence ou que la valeur relative à l'altitude du patient est comprise entre la troisième valeur (A3) de référence et une quatrième valeur (A4) de référence supérieure à la troisième valeur de référence.

7. Dispositif selon la revendication 6, dans lequel l'unité de contrôle (100) est configurée pour, si le patient est immergé et que la valeur relative à l'altitude du patient est supérieure à la quatrième valeur de référence (A4), déterminer une commande de l'actionneur pour augmenter le volume de fluide dans l'élément gonflable.

8. Dispositif selon l'une des revendications 2 à 7, dans lequel l'unité de contrôle (100) est configurée pour déclencher la commande de l'actionneur après l'écoulement d'une durée déterminée pendant laquelle la valeur relative à l'altitude du patient est supérieure à la première valeur de référence ou, si le patient est immergé, la valeur relative à l'altitude du patient est inférieure à une troisième valeur de référence.

9. Dispositif selon l'une des revendications 1 à 8, dans lequel le réservoir de fluide (5) comprend une partie fixe et une partie mobile (6, 7), l'actionneur (8) est couplé mécaniquement à ladite partie mobile (6, 7) de sorte à déplacer linéairement ladite partie mobile par rapport à la partie fixe pour faire varier le volume du réservoir (5) et ledit au moins un capteur comprend un capteur (101) agencé dans le boîtier (1), lié mécaniquement à l'actionneur (8) et/ou à la partie mobile (6, 7) du réservoir, agencé de sorte à mesurer une force de traction et/ou de compression dans la direction de déplacement de la partie mobile du réservoir, ladite force mesurée résultant au moins de :
- la force exercée par la partie mobile du réservoir liée à la pression dans le circuit fluidique, et
- la force exercée sur la partie mobile du réservoir liée à la pression de gaz dans le boîtier,
l'unité de contrôle étant configurée pour mesurer la pression de fluide dans le circuit fluidique à partir de ladite force mesurée.

10. Dispositif selon l'une des revendications 1 à 9, dans lequel ledit au moins un capteur comprend un capteur (102) de pression de gaz dans le boîtier (1) ou un altimètre (103) agencé dans le boîtier (1) et configuré pour mesurer une valeur relative à l'altitude du patient.

11. Dispositif selon l'une des revendications 1 à 10, comprenant en outre un dispositif de commande (9) extérieur au corps du patient et adapté pour être porté par le patient, ledit dispositif de commande (9) comprenant un capteur (90) de pression atmosphérique et étant adapté pour communiquer à l'unité de contrôle (100) la pression mesurée par ledit capteur de pression atmosphérique pour déterminer une valeur relative à l'altitude du patient.

12. Dispositif selon la revendication 9 en combinaison avec l'une des revendications 10 ou 11, dans lequel l'unité de contrôle est configurée pour détecter l'écart d'altitude à partir des données de mesure desdits capteurs de force (101), de pression (102, 90) ou de l'altimètre (103).

13. Dispositif selon la revendication 12, dans lequel l'unité de contrôle est configurée pour détecter l'écart d'altitude à partir des données de mesure d'un premier capteur parmi lesdits capteurs de force (101), de pression (102, 90) et l'altimètre (103) et pour confirmer ledit écart d'altitude détecté à partir des données de mesure d'un autre capteur parmi lesdits capteurs de force (101), de pression (102, 90) et l'altimètre (103).

14. Dispositif selon l'une des revendications 1 à 13, dans lequel l'unité de contrôle (100) est configurée pour détecter l'écart d'altitude à des intervalles réguliers compris entre 1 et 300 minutes, de préférence compris entre 5 et 60 minutes, encore de préférence compris entre 5 et 15 minutes.

15. Dispositif selon l'une des revendications 1 à 14, dans lequel l'élément gonflable (3) est une manchette occlusive adaptée pour être agencée autour d'un conduit anatomique afin d'obturer sélectivement ledit conduit anatomique, ledit dispositif étant configuré pour être implanté dans un corps humain ou animal pour obturer sélectivement au moyen de la manchette, un conduit anatomique choisi parmi au moins : un urètre, un conduit gastrique, un colon et un rectum.

## Patentansprüche

1. Medizinische Vorrichtung, umfassend:
- ein dichtes Gehäuse (1), das ein Gas enthält und geeignet ist, in einen Körper eines menschlichen oder tierischen Patienten implantiert zu werden,
- ein aufblasbares Element (3), das geeignet ist, außerhalb des Gehäuses in den Körper des Patienten implantiert zu werden,
- einen Fluidkreislauf, der einen Fluidbehälter (5) mit einem variablen Fluidvolumen, der in dem Gehäuse eingerichtet ist, und einen Schlauch (2) umfasst, der eine Fluidverbindung zwischen dem Behälter und dem aufblasbaren Element herstellt,
- einen Aktuator (8), der in dem Gehäuse eingerichtet und mechanisch mit dem Fluidbehälter gekoppelt ist, um das Fluidvolumen in dem Behälter selektiv zu ändern,
- eine Steuereinheit (100), die ausgelegt ist, um den Aktuator derart zu steuern, dass Fluid zwischen dem Behälter und dem aufblasbaren Element übertragen wird,
- mindestens einen Sensor (101, 102, 103, 90), der geeignet ist, einen absoluten Fluiddruck in dem Fluidkreislauf, eine auf den Behälter ausgeübte Kraft, einen Gasdruck in dem Gehäuse, einen atmosphärischen Druck oder einen Wert, der sich auf eine Höhe des Patienten bezieht, zu messen,
wobei die medizinische Vorrichtung **dadurch gekennzeichnet ist, dass** die Steuereinheit (100) ferner ausgelegt ist, um:
- ausgehend von einer Messung des Sensors, während der Aktuator inaktiv ist, eine Abweichung zwischen einem Wert (A), der sich auf eine Höhe des Patienten bezieht, und einem Referenzwert (A1, A2, A3, A4) zu erfassen und
- in Abhängigkeit von der Abweichung eine Steuerung des Aktuators (8) derart zu bestimmen, dass der Fluiddruck in dem Fluidkreislauf gesteuert wird.

2. Vorrichtung nach Anspruch 1, wobei die Steuereinheit (100) ausgelegt ist, um, wenn der Wert (A), der sich auf die Höhe des Patienten bezieht, größer als ein erster Referenzwert (A1) ist, den Aktuator zu steuern, um das Fluidvolumen in dem aufblasbaren Element zu reduzieren.

3. Vorrichtung nach Anspruch 2, wobei die Steuereinheit (100) ferner ausgelegt ist, um das reduzierte Fluidvolumen in dem aufblasbaren Element beizubehalten, solange der Wert (A), der sich auf die Höhe des Patienten bezieht, größer ist als der erste Referenzwert (A1) oder der Wert (A), der sich auf die Höhe des Patienten bezieht, zwischen dem ersten Referenzwert (A1) und einem zweiten Referenzwert (A2) liegt, der kleiner ist als der erste Referenzwert (A1).

4. Vorrichtung nach Anspruch 3, wobei die Steuereinheit (100) ausgelegt ist, um, wenn der Wert (A), der sich auf die Höhe des Patienten bezieht, kleiner ist als der zweite Referenzwert (A2), eine Steuerung des Aktuators zu bestimmen, um das Fluidvolumen in dem aufblasbaren Element zu erhöhen.

5. Vorrichtung nach einem der Ansprüche 1 bis 4, wobei die Steuereinheit (100) ausgelegt ist, um, wenn der Patient eingetaucht ist und der Wert (A) , der sich auf die Höhe des Patienten bezieht, kleiner ist als ein dritter Referenzwert (A3), den Aktuator zu steuern, um das Fluidvolumen in dem aufblasbaren Element zu reduzieren.

6. Vorrichtung nach Anspruch 5, wobei die Steuereinheit (100) ferner ausgelegt ist, um das reduzierte Fluidvolumen in dem aufblasbaren Element aufrechtzuerhalten, solange der Patient eingetaucht ist und der Wert, der sich auf die Höhe des Patienten bezieht, kleiner ist als der dritte Referenzwert (A3) oder der Wert, der sich auf die Höhe des Patienten bezieht, zwischen dem dritten Referenzwert (A3) und einem vierten Referenzwert (A4) liegt, der größer als der dritte Referenzwert ist.

7. Vorrichtung nach Anspruch 6, wobei die Steuereinheit (100) ausgelegt ist, um, wenn der Patient eingetaucht ist und der Wert, der sich auf die Höhe des Patienten bezieht, größer ist als der vierte Referenzwert (A4), eine Steuerung des Aktuators zu bestimmen, um das Fluidvolumen in dem aufblasbaren Element zu erhöhen.

8. Vorrichtung nach einem der Ansprüche 2 bis 7, wobei die Steuereinheit (100) ausgelegt ist, um die Steuerung des Aktuators nach Ablauf einer bestimmten Zeitdauer auszulösen, während der der Wert, der sich auf die Höhe des Patienten bezieht, größer ist als der erste Referenzwert oder, wenn der Patient eingetaucht ist, der Wert, der sich auf die Höhe des Patienten bezieht, kleiner ist als ein dritter Referenzwert.

9. Vorrichtung nach einem der Ansprüche 1 bis 8, wobei der Fluidbehälter (5) einen festen und einen beweglichen Teil (6, 7) umfasst, der Aktuator (8) mechanisch mit dem beweglichen Teil (6, 7) derart gekoppelt ist, dass der bewegliche Teil relativ zu dem festen Teil linear bewegt wird, um das Volumen des Behälters (5) zu ändern, und der mindestens eine Sensor einen in dem Gehäuse (1) eingerichteten Sensor (101) umfasst, der mechanisch mit dem Aktuator (8) und/oder dem beweglichen Teil (6, 7) des Behälters verbunden ist, so eingerichtet ist, dass eine Zug- und/oder Druckkraft in der Bewegungsrichtung des beweglichen Teils des Behälters gemessen wird, wobei die gemessene Kraft mindestens resultiert aus:
- der von dem beweglichen Teil des Behälters ausgeübten Kraft, die mit dem Druck im Fluidkreislauf verbunden ist, und
- der auf den beweglichen Teil des Behälters ausgeübten Kraft, die mit dem Gasdruck im Gehäuse verbunden ist,
wobei die Steuereinheit ausgelegt ist, um den Fluiddruck im Fluidkreislauf anhand der gemessenen Kraft zu messen.

10. Vorrichtung nach einem der Ansprüche 1 bis 9, wobei der mindestens eine Sensor einen Drucksensor (102) des Gases in dem Gehäuse (1) oder einen Höhenmesser (103) umfasst, der in dem Gehäuse (1) angeordnet und ausgelegt ist, um einen Wert, der sich auf die Höhe des Patienten bezieht, zu messen.

11. Vorrichtung nach einem der Ansprüche 1 bis 10, ferner umfassend eine Steuervorrichtung (9) außerhalb des Körpers des Patienten, die geeignet ist, von dem Patienten getragen zu werden, wobei die Steuervorrichtung (9) einen Sensor (90) des atmosphärischen Drucks umfasst und geeignet ist, der Steuereinheit (100) den vom atmosphärischen Drucksensor gemessenen Druck zu kommunizieren, um einen Wert zu bestimmen, der sich auf die Höhe des Patienten bezieht.

12. Vorrichtung nach Anspruch 9 in Kombination mit einem der Ansprüche 10 oder 11, wobei die Steuereinheit ausgelegt ist, um die Höhenabweichung ausgehend von den Messdaten der Sensoren (101) für Kraft, Druck (102, 90) oder des Höhenmessers (103) zu erfassen.

13. Vorrichtung nach Anspruch 12, wobei die Steuereinheit ausgelegt ist, um die Höhenabweichung ausgehend von den Messdaten eines ersten Sensors von den Sensoren (101) für Kraft, Druck (102, 90) und des Höhenmessers (103) zu erfassen und um die Höhenabweichung, die ausgehend von den Messdaten eines anderen Sensors von den Sensoren (101) für Kraft, Druck (102, 90) und des Höhenmessers (103) erfasst wurden, zu bestätigen.

14. Vorrichtung nach einem der Ansprüche 1 bis 13, wobei die Steuereinheit (100) ausgelegt ist, um die Höhenabweichung in regelmäßigen Intervallen zwischen 1 und 300 Minuten, vorzugsweise zwischen 5 und 60 Minuten, weiter vorzugsweise zwischen 5 und 15 Minuten, zu erfassen.

15. Vorrichtung nach einem der Ansprüche 1 bis 14, wobei das aufblasbare Element (3) eine Okklusionsmanschette ist, die geeignet ist, um um einen anatomischen Gang herum angeordnet zu werden, um den anatomischen Gang selektiv zu verschließen, wobei die Vorrichtung ausgelegt ist, um in einen menschlichen oder tierischen Körper implantiert zu werden, um mithilfe der Manschette selektiv einen anatomischen Gang zu verschließen, der zumindest ausgewählt ist aus: einer Harnröhre, einem Magengang, einem Dickdarm und einem Rektum.

## Claims

1. Medical device comprising :
- a sealed, gas-containing housing (1) adapted to be implanted in a human or animal patient's body,
- an inflatable element (3) adapted to be implanted in the patient's body outside the housing,
- a fluid circuit comprising a fluid reservoir (5) with a variable fluid volume arranged in the housing and a tube (2) providing a fluid connection between said reservoir and said inflatable element,
- an actuator (8) arranged in the housing and mechanically coupled to the fluid reservoir to selectively vary the volume of fluid in said reservoir,
- a control unit (100) configured to control the actuator so as to transfer fluid between the reservoir and the inflatable element,
- at least one sensor (101, 102, 103, 90) adapted to measure an absolute fluid pressure in the fluidic circuit, a force exerted on the reservoir, a gas pressure in the housing, an atmospheric pressure or a value relative to a patient's altitude,
said medical device being **characterized in that** said control unit (100) is further configured to:
- from a measurement of said sensor while the actuator is inactive, detect a deviation between a value (A) relating to a patient's altitude and a reference value (A1, A2, A3, A4) and
- determine, as a function of said deviation, control of the actuator (8) so as to control the fluid pressure in the fluid circuit.

2. Device according to claim 1, wherein the control unit (100) is configured so as, if the value (A) relating to the patient's altitude is greater than a first reference value (A1), to control the actuator to reduce the volume of fluid in the inflatable element.

3. Device according to claim 2, wherein the control unit (100) is further configured to maintain said reduced volume of fluid in the inflatable element as long as the value (A) relating to the patient's altitude is greater than the first reference value (A1) or the value (A) relating to the patient's altitude is between the first reference value (A1) and a second reference value (A2) lower than the first reference value (A1).

4. Device according to claim 3, wherein the control unit (100) is configured so as, if the value (A) relating to the patient's altitude is less than the second reference value (A2), to determine a command to the actuator to increase the volume of fluid in the inflatable element.

5. Device according to one of claims 1 to 4, wherein the control unit (100) is configured so as, if the patient is immersed and the value (A) relating to the patient's altitude is less than a third reference value (A3), to control the actuator to reduce the volume of fluid in the inflatable element.

6. Device according to claim 5, wherein the control unit (100) is further configured to maintain said reduced volume of fluid in the inflatable element as long as the patient is submerged and the value relating to the patient's altitude is less than the third reference value (A3) or the value relating to the patient's altitude is between the third reference value (A3) and a fourth reference value (A4) greater than the third reference value.

7. Device according to claim 6, wherein the control unit (100) is configured so as, if the patient is immersed and the value relating to the patient's altitude is greater than the fourth reference value (A4), to determine a command for the actuator to increase the volume of fluid in the inflatable element.

8. Device according to one of claims 2 to 7, wherein the control unit (100) is configured to trigger actuation of the actuator after the elapse of a specified time during which the value relating to the patient's altitude is greater than the first reference value or, if the patient is submerged, the value relating to the patient's altitude is less than a third reference value.

9. Device according to one of claims 1 to 8, wherein the fluid reservoir (5) comprises a fixed and a movable part (6, 7), the actuator (8) is mechanically coupled to said movable part (6, 7) so as to linearly displace said movable part relative to the fixed part to vary the volume of the reservoir (5) and said at least one sensor comprises a sensor (101) arranged in the housing (1), mechanically linked to the actuator (8) and/or to the movable part (6, 7) of the tank, arranged so as to measure a tensile and/or compressive force in the direction of displacement of the movable part of the tank, said measured force resulting at least from :
- the force exerted by the moving part of the reservoir in relation to the pressure in the fluid circuit, and
- the force exerted on the moving part of the reservoir by the gas pressure in the housing,
the control unit being configured to measure the fluid pressure in the fluid circuit from said measured force.

10. Device according to one of claims 1 to 9, wherein said at least one sensor comprises a gas pressure sensor (102) in the housing (1) or an altimeter (103) arranged in the housing (1) and configured to measure a value relating to the patient's altitude.

11. Device according to any of claims 1 to 10, further comprising a control device (9) external to the patient's body and adapted to be worn by the patient, said control device (9) comprising an atmospheric pressure sensor (90) and being adapted to communicate to the control unit (100) the pressure measured by said atmospheric pressure sensor to determine a value relating to the patient's altitude.

12. Device according to claim 9 in combination with one of claims 10 or 11, wherein the control unit is configured to detect the altitude deviation from the measurement data of said force sensors (101), pressure sensors (102, 90) or altimeter (103).

13. Device according to claim 12, wherein the control unit is configured to detect the altitude deviation from the measurement data of a first of said force (101), pressure (102, 90) and altimeter (103) sensors and to confirm said detected altitude deviation from the measurement data of another of said force (101), pressure (102, 90) and altimeter (103) sensors.

14. Device according to one of claims 1 to 13, wherein the control unit (100) is configured to detect the altitude deviation at regular intervals between 1 and 300 minutes, preferably between 5 and 60 minutes, even more preferably between 5 and 15 minutes.

15. Device according to any one of claims 1 to 14, wherein the inflatable element (3) is an occlusive cuff adapted to be arranged around an anatomical duct to selectively occlude said anatomical duct, said device being configured to be implanted in a human or animal body to selectively occlude by means of the cuff, an anatomical duct selected from at least: a urethra, a gastric duct, a colon and a rectum.
